(19) Europäisches Patentamt
European Patent Office
Office européen des brevets

(11) Veröffentlichungsnummer: **0 180 115**
**A2**

# (12) EUROPÄISCHE PATENTANMELDUNG

(21) Anmeldenummer: **85113278.7**

(22) Anmeldetag: **19.10.85**

(51) Int. Cl.⁴: **C 07 D 249/12**, C 07 D 401/04, A 61 K 31/41

(30) Priorität: **27.10.84 DE 3439450**

(43) Veröffentlichungstag der Anmeldung: **07.05.86 Patentblatt 86/19**

(84) Benannte Vertragsstaaten: **AT BE CH DE FR GB IT LI LU NL SE**

(71) Anmelder: **BOEHRINGER INGELHEIM KG, D-6507 Ingelheim am Rhein (DE)**
(84) Benannte Vertragsstaaten: **BE CH DE FR IT LI LU NL SE AT**

(71) Anmelder: **Boehringer Ingelheim International G.m.b.H, D-6507 Ingelheim am Rhein (DE)**
(84) Benannte Vertragsstaaten: **GB**

(72) Erfinder: **Weber, Karl-Heinz, Dr., Kaiser-Karl-Strasse 11, D-6535 Gau-Algesheim (DE)**
Erfinder: **Hinzen, Dieter, Prof. Dr., Konrad-Adenauer Strasse 26, D-6501 Zornheim (DE)**
Erfinder: **Kuhn, Franz Josef, Dr., Beethovenstrasse 11, D-6535 Gau-Algesheim (DE)**
Erfinder: **Lehr, Erich, Dr., In der Toffel 5, D-6531 Waldalgesheim (DE)**
Erfinder: **Frölke, Wilhelm, Dr., Matthias-Grünewald-Strasse 1, D-6507 Ingelheim/Rhein (DE)**
Erfinder: **Tröger, Wolfgang, Dr., St. Jakobus-Strasse 25, D-6534 Stromberg (DE)**
Erfinder: **Ensinger, Helmut, Dr., Im Herrengarten 10, D-6501 Wackernheim (DE)**
Erfinder: **Walther, Gerhard, Dr., Pfarrer-Heberer-Strasse 37, D-6530 Bingen/Rhein (DE)**
Erfinder: **Harreus, Albrecht, Dr., Sandstrasse 1, D-6507 Ingelheim/Rhein (DE)**

(54) **1,2,4-Triazolo-carbamate und ihre Säureadditionssalze, Verfahren zu ihrer Herstellung und Arzneimittel.**

(57) Neue 1,2,4-Triazolo-carbamate der allgemeinen Formel

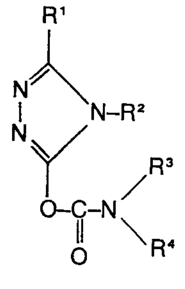

I

worin $R^1$, $R^2$, $R^3$ und $R^4$ die in der Beschreibung angegebene Bedeutung besitzen, sind Acetylcholinesterase-Hemmer und können als Arzneimittel zur Behandlung der Dementia senilis eingesetzt werden.

EP 0 180 115 A2

Die Erfindung betrifft neue 1,2,4-Triazolo-carbamate und deren Säureadditionssalze, Verfahren zu ihrer Herstellung und Arzneimittel, die diese Verbindungen enthalten.

Die neuen Verbindungen vermögen der Einschränkung des Kurzzeitgedächtnisses nach Gabe von muscarinen cholinergen Antagonisten entgegenzuwirken.
Bei der Dementia senilis vom Alzheimer-Typ, die mit einer Verarmung von Acetylcholin im Zentralnervensystem einhergeht, können die neuen Verbindungen als potentielle Arzneimittel zur Behandlung dieses Krankheitsbildes eingesetzt werden, da sie die zentrale Acetylcholinesterase langanhaltend aber reversibel hemmen.
Bekannte Acetylcholinesterase-Hemmer z.B. Alkylphosphate; Physostigmin; Neostigmin; Pyridostigmin; u.a., weisen erhebliche periphere Nebenwirkungen auf und sind sehr toxisch, teilweise nicht hirngängig, entweder zu kurz oder zu lang wirkend, so daß eine therapeutische Anwendung beim Menschen sehr erschwert bzw. unmöglich gemacht wird.

Gegenstand der Erfindung sind neue 1,2,4-Triazolo-carbamate der allgemeinen Formel

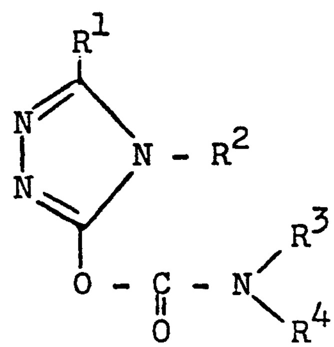

I

worin

$R^1$ Wasserstoff, eine verzweigte oder lineare Alkylgruppe mit 1 bis 8 Kohlenstoffatomen, einen 3- bis 6-gliedrigen carbocyclischen Ring, einen Benzyl- oder Phenethylrest;

$R^2$ einen Phenyl- oder Pyridinylrest, der ein- oder zweifach durch Halogen, Methyl, Methoxy oder Trifluormethyl substituiert sein kann;

$R^3$ und $R^4$, die gleich oder verschieden sein können, Wasserstoff, eine verzweigte oder lineare Alkylgruppe mit 1 bis 6 Kohlenstoffatomen, eine Cycloalkylgruppe mit 3 bis 6 Kohlenstoffatomen, einen, gegebenenfalls durch Halogen, Methoxy oder Trifluormethyl oder eine geradkettige oder verzweigte Alkylgruppe mit 1 bis 6 Kohlenstoffatomen substituierten Arylrest oder Heteroarylrest, $R^3$ und $R^4$ zusammen mit dem Stickstoffatom einen gesättigten, gegebenenfalls durch einen oder mehrere linear oder verzweigte Alkylgruppen mit 1 bis 4 Kohlenstoffatomen substituierten 5- oder 6-Ring, der als weiteres Heteroatom Stickstoff, Sauerstoff oder Schwefel enthalten kann und, im Fall von Stickstoff, dieser mit einer Alkylgruppe mit 1 bis 4 Kohlenstoffatomen oder einer Hydroxyalkylgruppe mit 1 bis 3 Kohlenstoffatomen substituiert sein kann, bedeutet und deren physiologisch unbedenkliche Säureadditionssalze.

Soweit nicht anderes angegeben, kann Halogen F, Cl, Br oder J bedeuten; bevorzugter Arylrest ist Phenyl, bevorzugte Heteroarylreste sind Pyridin und Pyrazin.

Die Verbindungen der allgemeinen Formel I können gegebenenfalls nach bekannten Methoden in ihre physiologisch unbedenklichen Säureadditionssalze überführt werden.

Als Säuren eignen sich hierfür sowohl anorganische Säuren, wie Halogenwasserstoffsäuren, Schwefelsäure, Phosphorsäure und Aminosulfonsäure, als auch organische Säuren wie Ameisensäure, Essigsäure, Propionsäure,

Milchsäure, Glykolsäure, Glukonsäure, Maleinsäure, Fumarsäure, Bernsteinsäure, Weinsäure, Benzoesäure, Salicylsäure, Zitronensäure, Ascorbinsäure, p-Toluolsulfonsäure oder Oxyäthansulfonsäure.

Bevorzugte Verbindungen der allgemeinen Formel I sind solche, in denen $R^1$ eine Methyl- oder Ethylgruppe; $R^2$ einen chlorsubstituierten Phenyl- oder Pyridinylrest; $R^3$ Wasserstoff, einen Methyl- oder Ethylrest, $R^4$ einen niederen Alkylrest mit 1 - 4 Kohlenstoffatomen, oder $R^3$ und $R^4$ zusammen mit dme Stickstoff einen N-methylsubstituierten Piperazinylrest bedeuten, wie z.B.

4-(5-Chlorpyridin-2-yl)-3-methyl-5-[(4-methylpiperazinyl)carbonyl]oxy-1,2,4-triazol,

4-(5-Chlorpyridin-2-yl)-3-methyl-5-[N-tert.-butylaminocarbonyl]oxy-1,2,4,-triazol,

4-(4- Chlorphenyl)-3-ethyl-5-[(4-methylpiperazinyl)-carbonyl]oxy-1,2,4-triazol,

4-(5-Chlorpyridin-2-yl)-3-methyl-5-[N,N-diethylaminocarbonyl]oxy-1,2,4-triazol,

4-(5-Chlorpyridin-2-yl)-3-methyl-5-[N-methylaminocarbonyl]oxy-1,2,4-triazol,

4-(5-Chlorpyridin-2-yl)-3-methyl-5-[N,N-dimethylaminocarbonyl]oxy-1,2,4-triazol,

4-(4-Chlorphenyl)-3-methyl-5-[(4-methylpiperazinyl)-carbonyl]oxy-1,2,4-triazol.

Gegenstand der Erfindung sind ferner Verfahren zur Herstellung der Verbindungen der allgemeinen Formel I.

Ausgehend von 5-Hydroxy-1,2,4-triazolen der allgemeinen Formel

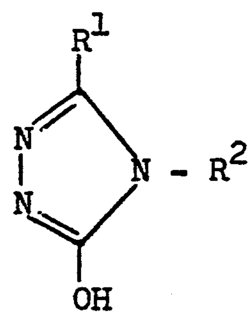

II,

worin
$R^1$ und $R^2$ die oben genannte Bedeutung haben, werden die Verbindungen der allgemeinen Formel I dadurch erhalten, daß 5-Hydroxytriazole der allgemeinen Formel II, gegebenenfalls unter Zusatz von Basen, mit einem Halogencarbonylamid der allgemeinen Formel

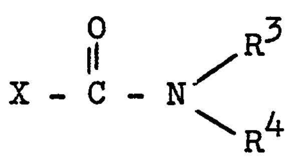

III,

worin
$R^3$ und $R^4$ die oben angegebene Bedeutung haben und X ein Halogen, bevorzugt Chlor, ist, zur Reaktion gebracht werden. Hierbei wird eine Verbindung der allgemeinen Formel II entweder mit einem Halogencarbonylamid der allgemeinen Formel III, zweckmäßigerweise unter Zusatz einer tertiären organischen Base, wie z.B. Triethylamin oder Pyridin, oder in Gegenwart einer anderen Base umgesetzt, oder aber Verbindungen der allgemeinen Formel II mit Basen in ihre Salze überführt

und diese mit einem Halogencarbonylamid der allgemeinen Formel III umgesetzt. Im allgemeinen ist es nicht erforderlich, die Salze zu isolieren.

Als Basen werden bevorzugt Alkali- oder Erdalkalihydride, wie z.B. Natriumhydrid oder Kalziumhydrid; Alkoholate, wie z.B. Natriummethanolat, Natriumethanolat, Natriumisopropylat, Kaliumtertiärbutanolat oder auch Aluminiumisopropylat eingesetzt.

Die Reaktion findet vorzugsweise unter Verwendung eines organischen Lösungsmittels zwischen 0°C und dem Siedepunkt des Lösungsmittels unter Feuchtigkeitsausschluß statt. Geeignete Lösungsmittel sind Ether, cyclische Ether, wie z.B. Tetrahydrofuran oder Dioxan, geeignete halogenierte Kohlenwasserstoffe, wie z.B. Methylenchlorid; Acetonitril, Dimethylformamid, Dimethylsulfoxid, Toluol oder Benzol.

Ein weiteres Verfahren zur Herstellung von Verbindungen der allgemeinen Formel I besteht darin, daß Verbindungen der allgemeinen Formel II, gegebenenfalls unter Zusatz von Basen mit einem geeigneten Halogenkohlensäureester der allgemeinen Formel

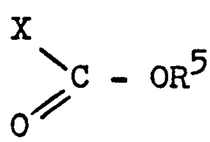

IV,

worin
X wie oben definiert und $R^5$ eine als Austrittsgruppe geeignete Alkyl- oder Arylgruppe bedeutet, zur Reaktion gebracht und anschließend mit einem primären oder sekundären Amin der allgemeinen Formel

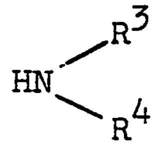

V,

worin
$R^3$ und $R^4$ die oben genannte Bedeutung aufweisen, umgesetzt wird.

Hierbei wird eine Verbindung der allgemeinen Formel II mit einem geeigneten Halogenkohlensäureester der allgemeinen Formel IV, worin $R^5$ vorzugsweise eine Phenyl-, Nitrophenyl- oder Benzylgruppe bedeutet, gegebenenfalls unter Zusatz einer tertiären organischen Hilfsbase, wie z.B. Triethylamin oder Pyridin, oder einer anderen Base umgesetzt, oder aber die Verbindung der allgemeinen Formel II mit Basen in ihre Salze überführt und mit Halogenkohlensäureester umsetzt.

Bevorzugte Basen sind Alkali- oder Erdalkalihydride, wie z.B. Natriumhydrid oder Kalziumhydrid; Alkoholate, wie z.B. Natriummethanolat, Natriumethanolat, Natriumisopropylat, Kaliumtertiärbutanolat oder Aluminiumisopropylat.

Die Umsetzung von II bzw. dessen Salzen mit IV findet vorzugsweise in einem inerten organischen Lösungsmittel, wie z.B. Ether, cyclische Ether, wie z.B. Tetrahydrofuran oder Dioxan, geeigneten halogenierten Kohlenwasserstoffen, wie z.B. Methylenchlorid, Acetonitril, Dimethylformamid, Dimethylsulfoxid, Toluol oder Benzol, zwischen $0^{o}C$ und dem Siedepunkt des Lösungsmittels unter Feuchtigkeitsausschluß statt.
Die bei der Umsetzung als Zwischenprodukte entstehenden Triazolokohlensäureester können isoliert oder aber in situ weiter umgesetzt werden. Man erhält bei der Behandlung mit primären oder sekundären Aminen die entsprechenden Endprodukte der allgemeinen Formel I.

Die Reaktion kann in einem der oben genannten Lösungsmittel oder aber auch ohne Lösungsmittel in einem Überschuß des Amins durchgeführt werden, die Temperaturen liegen etwa zwischen -20°C und dem Siedepunkt des Lösungsmittels, wobei die einzelnen Reaktionsbedingungen von der Basizität und dem Siedepunkt des Amins abhängen. Bei niedrig siedenden Aminen muß die Reaktion unter Umständen im Autoklaven erfolgen.

Ein weiteres Verfahren zur Herstellung von Verbindungen der allgemeinen Formel I ist die Umsetzung von Verbindungen der allgemeinen Formel II unter Zusatz von Basen mit Carbamidsäureestern der allgemeinen Formel

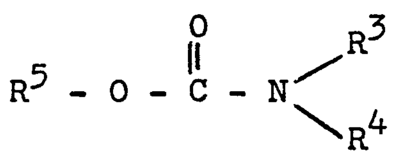

VI

worin
$R^3$, $R^4$ und $R^5$ die oben genannte Bedeutung besitzen.

Hierbei wird das Triazol II unter Zusatz einer Base in sein entsprechendes Salz überführt und dann mit dem Ester VI umgesetzt. Eine Isolierung des Salzes ist nicht unbedingt erforderlich. Geeignete inerte Lösungsmittel sind Ether, cyclische Ether, wie z.B. Tetrahydrofuran oder Dioxan, geeignete halogenierte Kohlenwasserstoffe, wie z.B. Methylenchlorid; Acetonitril, Dimethylformamid, Dimethylsulfoxid, Toluol oder Benzol.

Als Basen werden bevorzugt Alkali- oder Erdalkalihydride, wie z.B. Natriumhydrid oder Kalziumhydrid, Alkoholate, wie z.B. Natriummethanolat, Natriumethanolat, Natriumisopropylat, Kaliumtertiärbutanolat oder Aluminiumisopropylat verwendet. Bevorzugt wird die Reaktion unter Feuchtigkeitsausschluß zwischen 0°C und dem Siedepunkt des Lösungsmittels durchgeführt.

Verbindungen der allgemeinen Formel I mit $R^3$ gleich Wasserstoff erhält man durch Umsetzung von Verbindungen der allgemeinen Formel II, gegebenenfalls unter Zusatz von Basen,mit geeigneten Isocyanaten der allgemeinen Formel

$$O = C = N - R^4 \qquad VII,$$

worin
$R^4$ die oben genannte Bedeutung aufweist.

Hierbei wird eine Verbindung der allgemeinen Formel II mit dem Isocyanat, gegebenenfalls unter Zusatz einer tertiären organischen Hilfsbase, wie z.B. Triethylamin oder Pyridin,oder einer anderen Base umgesetzt, oder aber eine Verbindung der allgemeinen Formel II mit Basen in ihre Salze überführt und mit dem Isocyanat VII umgesetzt. Im allgemeinen ist es nicht notwendig, die Salze zu isolieren.

Die Umsetzungen werden vorzugsweise in inerten Lösungsmitteln, wie z.B. Ethern, cyclischen Ethern, wie z.B. Tetrahydrofuran, Dioxan, geeigneten halogenierten Kohlenwasserstoffen, wie z.B. Methylenchlorid; Acetonitril, Dimethylformamid, Dimethylsulfoxid, Benzol oder Toluol zwischen -50°C und dem Siedepunkt des Lösungsmittels unter Feuchtigkeitsausschluß durchgeführt.

Werden Verbindungen der allgemeinen Formel II unter Zusatz von Alkoholaten in ihre Salze überführt, können höher siedende Lösungsmittel, z.B. Dioxan, verwendet werden, um den dabei entstehenden Alkohol vor Zugabe der Carbamat bildenden Komponente, z.B. Chlorkohlensäureester,azeotrop abzudestillieren.

Die Ausgangsmaterialien für die vorstehend beschriebenen Verfahren sind zum Teil literaturbekannt oder können nach bekannten Verfahren hergestellt werden.

Beispielsweise lassen sich 5-Hydroxy-1,2,4-triazole, in denen $R^2$ ein Pyridylrest ist, nach folgendem Syntheseverfahren herstellen.

Die Umsetzung von substituierten 2-Aminopyridinen mit Orthoameisensäureethylester führt zu den entsprechenden Imidoethylestern (1). Der Ringschluß zu einem 1,2,4-Triazolderivat (2) erfolgt durch Erhitzen mit Essigsäurehydrazid in einem höhersiedenden Lösungsmittel, wie z.B. Diglyme. Anschließende Bromierung mit Brom oder N-Bromsuccinimid ergibt das Bromderivat (3), welches dann zu einem 5-Hydroxy-1,2,4-triazol der allgemeinen Formel II hydrolysiert werden kann.

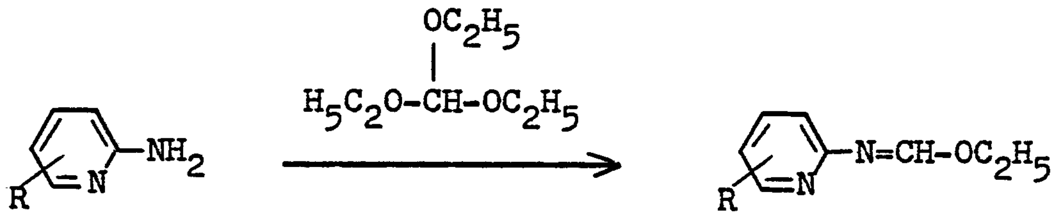

(1)

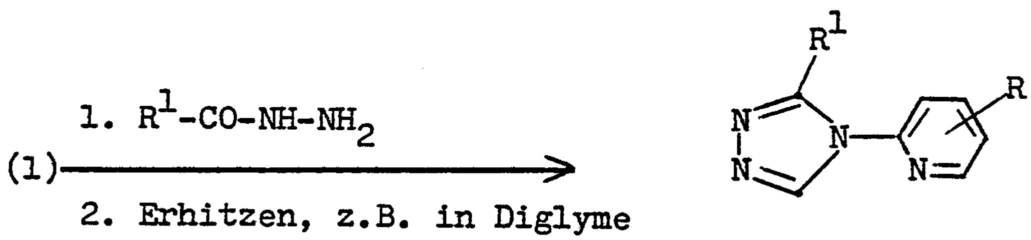
1. $R^1$-CO-NH-$NH_2$
(1)
2. Erhitzen, z.B. in Diglyme

(2)

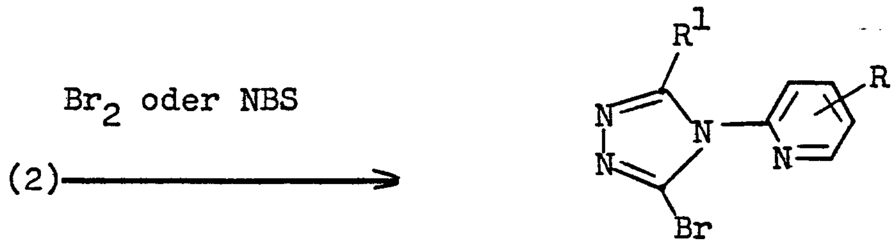
$Br_2$ oder NBS
(2)

(3)

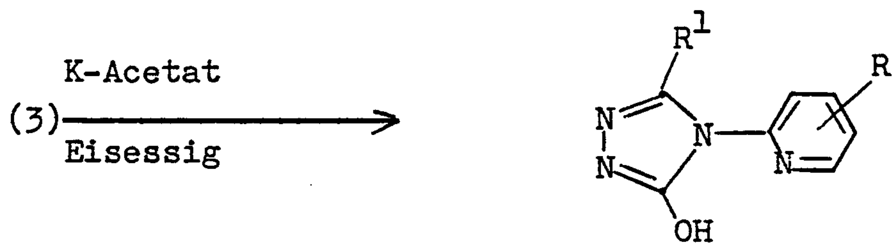
K-Acetat
(3)
Eisessig

II

Die neuen Verbindungen bewiesen in tierexperimentellen, pharmakologischen Untersuchungen eine

- reversible Hemmung der zentralen Acetylcholinesterase,
- Verbesserung der zentralnervösen, kortikalen Wach-Weckreaktion,
- signifikante Förderung des REM-Schlafanteils bei unverändertem Muster des Gesamtschlafs,
- Aktivierung der Entladungsfrequenz zentraler cholinerger Nervenzellen,
- Förderung von Lern- und Gedächtnisleistungen,
- Verbesserung des Übergangs von Inhalten des Kurzzeitin das Langzeitgedächtnis nach vorheriger Blockade durch den muscarin-cholinergen Antagonisten Scopolamin.

Im Vergleich zu bekannten Acetylcholinesterase-Hemmern weisen die erfindungsgemäßen Verbindungen eine sehr geringe Toxizität und keine störenden peripheren Nebenwirkungen auf.

Da die Verarmung des chemischen Überträgerstoffes Acetylcholin im Gehirn ein wesentliches Kennzeichen der senilen degenerativen Demenz vom Alzheimer-Typ darstellt, und dieses Krankheitsbild mit erheblicher Beeinträchtigung kognitiver und Gedächtnisleistungen einhergeht, können die neuen Verbindungen als Arzneimittel zur Behandlung des obigen Krankheitsbildes eingesetzt werden.

Die neuen Verbindungen können allein oder in Kombination mit anderen erfindungsgemäßen Wirkstoffen, gegebenenfalls auch in Kombination mit weiteren pharmakologisch aktiven Wirkstoffen, z.B. Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Die nachfolgenden Beispiele illustrieren die vorliegende Erfindung, ohne sie jedoch in ihrem Umfang zu beschränken:

Beispiel 1

4-(5-Chlorpyridin-2-yl)-3-methyl-5-[(4-methyl-piperazin-1-yl)carbonyl]oxy-1,2,4-triazol

50 g (0,24 Mol) 4(5- Chlorpyridin-2-yl)-5-hydroxy-3-methyl-1,2,4-triazol werden in 1 l absolutem Tetrahydrofuran gelöst bzw. suspendiert. Hierzu gibt man das mit Toluol entfettete Natriumhydrid aus 10,8 g einer 55%igen Natriumhydriddispersion und rührt 1 Stunde bei Raumtemperatur. Danach tropft man 39 g (0,24 Mol) frisch destilliertes 1-Chlorcarbonyl-4-methyl-piperazin ($Kp_{17}$: 120 - 124°C) hinzu und rührt unter Feuchtigkeitsausschluß weitere 5 Stunden. Die entstandene Suspension wird im Vakuum eingeengt und der Rückstand vorsichtig mit Wasser versetzt und neutralisiert. Die das Carbamat enthaltende Lösung wird mehrmals mit Methylenchlorid ausgeschüttelt und die mit Wasser gewaschene organische Phase nach dem Trocknen eingedampft. Der Rückstand wird mit Ether verrieben und man erhält 55 g (68 % d.Th.) der Titelverbindung als Kristalle vom Fp. 121 - 122°C.

12,5 g dieser Base löst man in 100 ml Methanol und versetzt heiß mit 4,3 g Fumarsäure. Beim Abkühlen kristallisiert das Semifumarat vom Fp. 173 - 175°C aus (Ausbeute: 17 g). Die Verbindung ist gut wasserlöslich; pH der Lösung 3,5.

Die Ausgangsverbindung, das 4-(5-Chlorpyridin-2-yl)-5-hydroxy-3-methyl-1,2,4-triazol, wird wie folgt erhalten:

a) 81,6 g (0,64 Mol) fein gemahlenes 2-Amino-5-chlorpyridin und 0,6 g seines Hydrochlorids werden mit 224 g (1,5 Mol) Orthoameisensäureethylester

im Ölbad auf 130 - 140°C erhitzt und hierbei das entstehende Ethanol abdestilliert. Dies nimmt 5 - 6 Stunden in Anspruch. Man fügt zu dem entstandenen festen Rückstand 96 g (1,3 Mol) Essigsäurehydrazid und 1,8 l Ethanol zu und kocht weitere 6 Stunden unter Rückfluß. Nach dem Abkühlen werden 82 g Kristalle vom Fp. 213 - 215°C erhalten.

b) 81,2 g (0,38 Mol) dieser Verbindung werden in 1,5 l Diglyme suspendiert und auf 120°C erhitzt. Dann fügt man 96 ml Pyridin hinzu und erhitzt 20 - 25 Stunden auf 140 - 145°C. 1,4 l Diglyme werden anschließend im Vakuum abdestilliert und der abgekühlte Rückstand mit 1,7 l Petrolether versetzt, wobei das Triazolderivat auskristallisiert. Man erhält nach Absaugen 62 g des gewünschten Triazols als Kristalle vom Fp. 128 - 130°C.

c) 61,7 g (0,32 Mol) dieser Substanz werden in 720 ml Methylenchlorid gelöst und mit 26 ml (0,32 Mol) Pyridin versetzt. Unter Rühren fügt man bei Raumtemperatur 16,3 ml (0,32 Mol) Brom hinzu und rührt 5 - 7 Stunden nach. Das Reaktionsgemisch wird mit Wasser gewaschen. Anschließend wird die Methylenchloridphase getrocknet und eingedampft. Der Rückstand wird aus Essigester umkristallisiert und man erhält 41 g der Bromverbindung als Kristalle vom Fp. 150 - 153°C.

d) 21,6 g (0,08 Mol) der Bromverbindung werden in 200 ml Eisessig und 33,2 g Kaliumacetat 1 - 2 Stunden unter Rückfluß gekocht. Man kühlt ab, fügt 200 ml Wasser hinzu, saugt die ausgefallenen Kristalle ab und wäscht diese mit Wasser.
Ausbeute 14 g, Fp. 214 - 216°C.

Beispiel 2

4-(5-Chlorpyridin-2-yl)-3-methyl-5-(N-tert.-butylaminocarbonyl)oxy-1,2,4-triazol

19,6 g (0,09 Mol) 4-(5-Chlorpyridin-2-yl)-5-hydroxy-3-methyl-1,2,4-triazol werden in 600 ml absolutem Tetrahydrofuran suspendiert. Man erhitzt zum Sieden und fügt 20 g (0,2 Mol) tert.-Butylisocyanat hinzu, erhitzt 10 Minuten und gibt 4 ml einer 5%igen methanolischen Natriummethylatlösung zu dem Reaktionsgemisch. Es wird 24 Stunden unter Rückflußbedingungen gerührt, anschließend die Suspension eingedampft und der Rückstand in Methylenchlorid gelöst und die unlöslichen Bestandteile abgesaugt. Man wäscht die organische Phase nacheinander mit 2 N Salzsäure, mit Natriumhydrogencarbonat-Lösung und mit Wasser, trocknet mit Magnesiumsulfat und dampft im Vakuum ein. Nach der Zugabe von Ether konnten 25 g (87 d.Th.) der Titelverbindung als Kristalle vom Fp. 132 - 134°C isoliert werden.

Auf diese oder ähnliche Weise wurden auch folgende Carbamate erhalten:

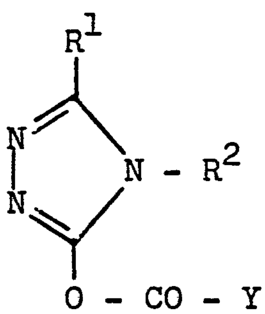

| Beispiel | $R^1$ | $R^2$ | Y | Fp. °C |
|---|---|---|---|---|
| 3 | H | 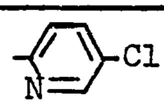 | 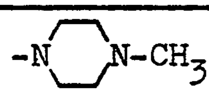 | 167 - 168 |
| 4 | 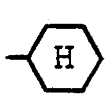 | 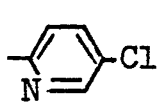 | 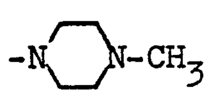 | 177 - 178 |

| Beispiel | $R^1$ | $R^2$ | Y | Fp. °C |
|---|---|---|---|---|
| 5 | $-CH_3$ | 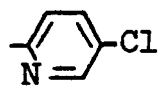 | H<br>$-N-CH_3$ | 155 - 156 |
| 6 | $-CH_3$ | 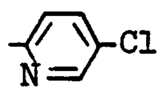 | 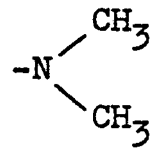 | 151 - 152 |
| 7 | $-CH_3$ | 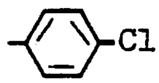 | 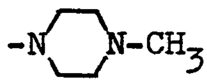 | 154 - 156 |
| 8 | $-CH_3$ | 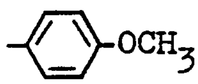 | 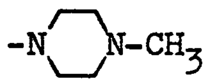 | 128 - 129 |
| 9 | 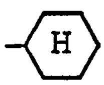 | 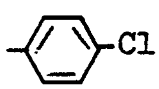 | 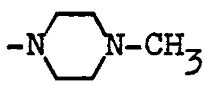 | Fu: 158 - 160 |
| 10 | 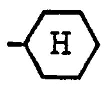 | 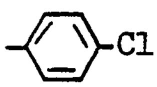 | 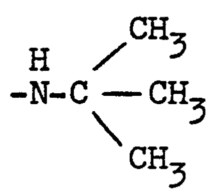 | 169 - 170 |
| 11 | 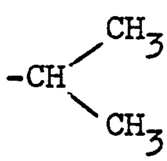 | 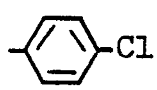 | 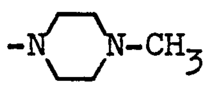 | Fu: 204 - 205 |
| 12 | 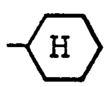 | 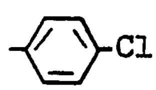 | H<br>$-N-CH_3$ | 186 - 187 |
| 13 | 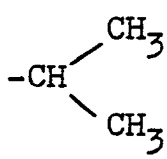 | 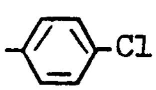 | 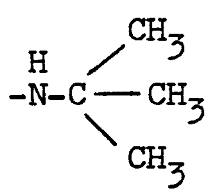 | 184 - 185 |
| 14 | 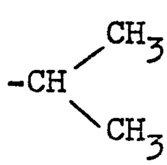 | 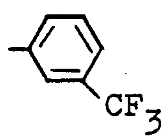 | 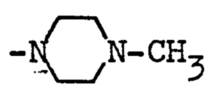 | Fu: 161 - 162 |
| 15 | 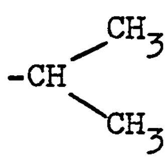 | 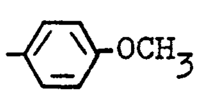 | 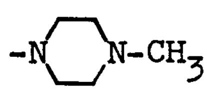 | Fu: 174 - 175 |

| Bei-spiel | $R^1$ | $R^2$ | Y | Fp. °C |
|---|---|---|---|---|
| 16 | $-CH_3$ | 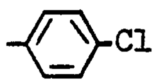 | 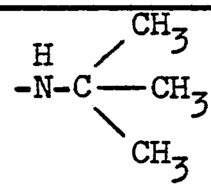 | 160 - 161 |
| 17 | $-CH_3$ | 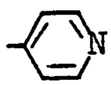 | 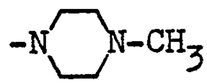 | Fu: 124 - 125 |
| 18 | $-CH_3$ | 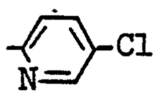 | 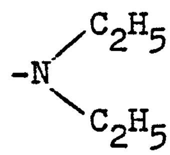 | 135 - 136 |
| 19 | $-CH_3$ | 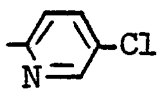 | 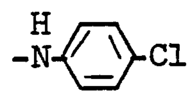 | 176 - 177 |
| 20 | $-CH_3$ | 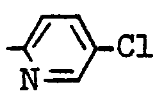 | 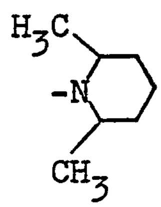 | 101 - 103 |
| 21 | $-CH_3$ | 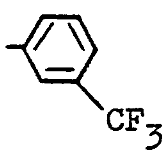 | 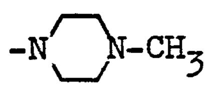 | 123 - 124 |
| 22 | $-CH_2-CH_3$ | 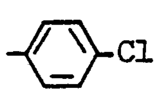 | 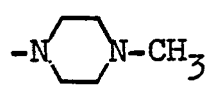 | 192 - 193 |

Fu = Fumarat

## Pharmazeutische Formulierungsbeispiele

A) Tabletten

| Tabletten | pro Tablette |
|---|---|
| Wirkstoff | 100 mg |
| Milchzucker (pulverisiert) | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B) Tabletten

| Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

C) Ampullen

| | | |
|---|---|---|
| 4-(5-Chlorpyridin-2-yl)-3-methyl-5-[(4-methyl-piperazin-1-yl)carbonyl]oxy-1,2,4-triazol x Fu | | 50,0 mg |
| Natriumchlorid | | 10,0 mg |
| bidestilliertes Wasser | q.s. ad | 1,0 ml |

Herstellung:

Der Wirkstoff und das Natriumchlorid werden in bidestilliertem Wasser gelöst und die Lösung in Ampullen steril abgefüllt.

D) Tropfen

| | | |
|---|---|---|
| 4-(5-Chlorpyridin-2-yl)-3-methyl-5-[(4-methyl-piperazin-1-yl)carbonyl]oxy-1,2,4-triazol x Fu | | 5,0 g |
| p-Hydroxybenzoesäuremethylester | | 0,1 g |
| p-Hydroxybenzoesäurepropylester | | 0,1 g |
| entmineralisiertes Wasser | q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

0180115

## Patentansprüche

1. Neue 1,2,4-Triazolo-carbamate der allgemeinen Formel

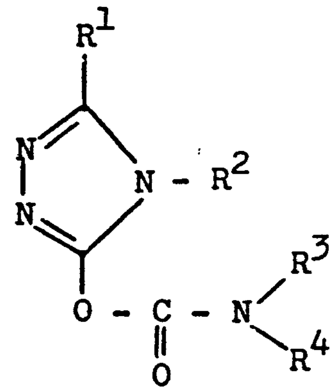

I

worin

$R^1$ Wasserstoff, eine verzweigte oder lineare Alkylgruppe mit 1 bis 8 Kohlenstoffatomen, einen 3- bis 6-gliedrigen carbocyclischen Ring, einen Benzyl- oder Phenethylrest;

$R^2$ einen Phenyl- oder Pyridinylrest, der ein- oder zweifach durch Halogen, Methyl, Methoxy oder Trifluormethyl substituiert sein kann;

$R^3$ und $R^4$, die gleich oder verschieden sein können, Wasserstoff, eine verzweigte oder lineare Alkylgruppe mit 1 bis 6 Kohlenstoffatomen, eine Cycloalkylgruppe mit 3 bis 6 Kohlenstoffatomen, einen, gegebenenfalls durch Halogen, Methoxy, Trifluormethyl oder eine geradkettige oder verzweigte Alkylgruppe mit 1 bis 6 Kohlenstoffatomen substituierten Arylrest oder Heteroarylrest,

$R^3$ und $R^4$ zusammen mit dem Stickstoffatom einen gesättigten, gegebenenfalls durch einen oder mehrere linear oder verzweigte Alkylgruppen mit 1 bis 4 Kohlenstoffatomen substituierten 5- oder 6-Ring, der als weiteres Heteroatom N, O oder S enthalten kann und, im Fall von N, dieser mit einer Alkylgruppe mit 1 bis 4 Kohlenstoffatomen oder einer Hydroxyalkylgruppe mit 1 bis 3 Kohlenstoffatomen substituiert sein kann, bedeutet und deren physiologisch unbedenkliche Säureadditionssalze.

2. Verbindungen der allgemeinen Formel I nach Anspruch 1, dadurch gekennzeichnet, daß
$R^1$ eine Methyl- oder Ethylgruppe;
$R^2$ einen chlorsubstituierten Phenyl- oder Pyridinylrest;
$R^3$ Wasserstoff, einen Methyl- oder Ethylrest;
$R^4$ einen niederen Alkylrest mit 1 bis 4 Kohlenstoffatomen, oder $R^3$ und $R^4$ zusammen mit dem Stickstoffatom einen N-methylsubstituierten Piperazinylrest bedeuten.

3. 4-(5-Chlorpyridin-2-yl)-3-methyl-5-[(4-methylpiperazinyl)carbonyl]oxy-1,2,4-triazol.

4. 4-(5- Chlorpyridin-2-yl)-3-methyl-5-[N-tert.-butylaminocarbonyl]oxy-1,2,4-triazol.

5. 4-(4-Chlorphenyl)-3-ethyl-5-[(4-methylpiperazinyl)-carbonyl]oxy-1,2,4-triazol.

6. 4-(5-Chlorpyridin-2-yl)-3-methyl-5-[N,N-diethylaminocarbonyl]oxy-1,2,4-triazol.

7. 4-(5-Chlorpyridin-2-yl)-3-methyl-5-[N-methylaminocarbonyl]oxy-1,2,4-triazol.

8. 4-(5-Chlorpyridin-2-yl)-3-methyl-5-[N,N-dimethylaminocarbonyl]oxy-1,2,4-triazol.

9. 4-(4-Chlorphenyl)-3-methyl-5-[(4-methylpiperazinyl)-carbonyl]oxy-1,2,4-triazol.

10. Verfahren zur Herstellung neuer Verbindungen der allgemeinen Formel

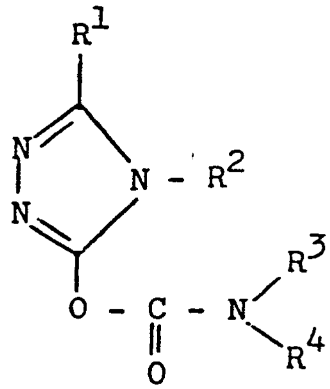

I

worin

$R^1$ Wasserstoff, eine verzweigte oder lineare Alkylgruppe mit 1 bis 8 Kohlenstoffatomen, einen 3- bis 6-gliedrigen carbocyclischen Ring, einen Benzyl- oder Phenethylrest;

$R^2$ einen Phenyl- oder Pyridinylrest, der ein- oder zweifach durch Halogen, Methyl, Methoxy oder Trifluormethyl substituiert sein kann;

$R^3$ und $R^4$, die gleich oder verschieden sein können, Wasserstoff, eine verzweigte oder lineare Alkylgruppe mit 1 bis 6 Kohlenstoffatomen, eine Cycloalkylgruppe mit 3 bis 6 Kohlenstoffatomen, einen, gegebenenfalls durch Halogen, Methoxy, Trifluormethyl oder eine geradkettige oder verzweigte Alkylgruppe mit 1 bis 6 Kohlenstoffatomen substituierten Arylrest oder Heteroarylrest,

$R^3$ und $R^4$ zusammen mit dem Stickstoffatom einen gesättigten, gegebenenfalls durch einen oder mehrere linear oder verzweigte Alkylgruppen mit 1 bis 4 Kohlenstoffatomen substituierten 5- oder 6-Ring, der als weiteres Heteroatom N, O oder S enthalten kann und, im Fall von N, dieser mit einer Alkylgruppe mit 1 bis 4 Kohlenstoffatomen oder einer Hydroxyalkylgruppe mit 1 bis 3 Kohlenstoffatomen substituiert sein kann, bedeutet,

dadurch gekennzeichnet, daß man Verbindungen der allgemeinen Formel

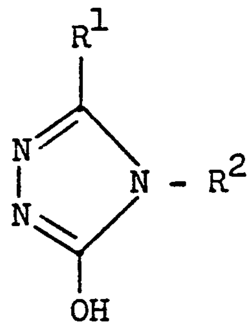

II

worin
$R^1$ und $R^2$ wie zuvor definiert, gegebenenfalls unter Zusatz von Basen,

a) mit einem Halogencarbonylamid der allgemeinen Formel

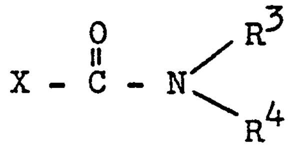

III

worin
$R^3$ und $R^4$ die oben genannte Bedeutung haben und X ein Halogen, bevorzugt Chlor, bedeutet,

b) oder mit einem Halogenkohlensäureester der allgemeinen Formel

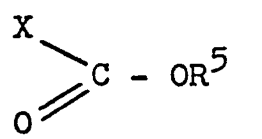

IV

worin
$R^5$ eine als Austrittsgruppe geeignete Alkyl- oder Arylgruppe bedeutet und X ein Halogen, bevorzugt Chlor, ist und anschließend mit einem primären oder sekundären Amin der allgemeinen Formel

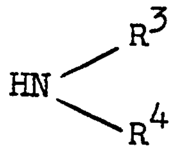

V

worin
$R^3$ und $R^4$ wie oben definiert sind,

c) oder mit einem Carbamidsäureester der allgemeinen Formel

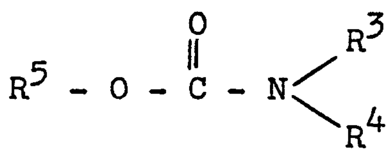

VI

worin
$R^3$, $R^4$ und $R^5$ wie oben definiert sind,

d) oder zur Herstellung solcher Verbindungen der allgemeinen Formel I, in denen $R^3$ Wasserstoff bedeutet, mit einem Isocyanat der allgemeinen Formel

$$O = C = N - R^4 \qquad \text{VII}$$

worin
$R^4$ wie oben definiert ist, umsetzt, und daß man gegebenenfalls ein so erhaltenes Endprodukt der allgemeinen Formel I auf üblichem Weg in ein physiologisch unbedenkliches Säureadditionssalz überführt.

11. Verfahren nach Anspruch 10, dadurch gekennzeichnet, daß Verbindungen der allgemeinen Formel II mit Basen in ihre Salze überführt und dann mit einer Verbindung der allgemeinen Formel III, IV, VI oder VII umgesetzt werden.

12. Pharmazeutische Zubereitungen, enthaltend als Wirkstoff eine oder mehrere Verbindungen der allgemeinen Formel I oder deren physiologisch verträgliche Säureadditionssalze in Kombination mit üblichen Hilfs- und/oder Trägerstoffen.

13. Verfahren zur Herstellung von pharmazeutischen Präparaten gemäß Anspruch 12, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

14. Methode zur Behandlung der Zustandsformen eingeschränkter cerebraler Leistungsfähigkeit mittels pharmazeutischer Zusammensetzungen gemäß Anspruch 12.